# EUROPÄISCHE PATENTSCHRIFT

(11) **EP 1 255 487 B1**
(45) Veröffentlichungstag und Bekanntmachung des Hinweises auf die Patenterteilung: **05.01.2005**
(21) Anmeldenummer: 00979323.3
(22) Anmeldetag: 18.12.2000
(51) Int. Cl.: A61B 6/08

(54) **LASERINDIKATOR**
LASER POINTER
POINTEUR LASER

(30) Priorität: 15.02.2000 DE 20002604 U
(43) Veröffentlichungstag der Anmeldung: 13.11.2002
(73) Patentinhaber: SYNTHES AG Chur, 7002 Chur (CH)
(72) Erfinder: SUHM, Norbert, 79576 Weil-Haltingen (DE); MESSMER, Peter, CH-4104 Oberwil (CH); REGAZZONI, Pietro, CH-4054 Basel (CH); HEHLI, Markus, CH-7276 Frauenkirch (CH); MÜLLER, Paul, CH-4125 Riehen (CH)
(74) Vertreter: Lusuardi, Werther Giovanni, Dr.
(86) Internationale Anmeldenummer: PCT/CH2000/000668
(87) Internationale Veröffentlichungsnummer: WO 2001/060259

(56) Entgegenhaltungen:
- US-A- 5 537 453
- US-A- 5 661 775
- US-A- 5 732 703

## Beschreibung

Die Erfindung bezieht sich auf eine Vorrichtung für ein chirurgisches Navigationssystem zur Definition der Lage einer von einem Operateur bestimmbaren Geraden innerhalb eines dreidimensionalen Koordinatensystems in einem Operationsraum gemäss dem Oberbegriff des Patentanspruchs 1.

Immer häufiger werden in chirurgischen Operationsräumen bei der Behandlung von Knochenfrakturen sogenannte Computerassistierte Chirurgiesysteme (CAS: Computer Assisted Surgery System) oder Chirurgische Navigationssysteme eingesetzt. Diese Geräte ermöglichen die Anwendung bildgestützter, minimalinvasiver Operationstechniken indem sie die örtliche Vermessung von chirurgischen Instrumenten und Geräten innerhalb eines im Operationsraum festen, dreidimensionalen Koordinatensystems erlauben. Damit lassen sich beispielsweise medizinische Roboter oder auch bewegbare Röntgengeräte, insbesondere solche, die auf einem im Raum bewegbaren C-förmigen Bogen an einem Ende eine Röntgenstrahlenquelle und am anderen Ende einen Röntgenstrahlenempfänger (im folgenden kurz C-Bogen genannt) aufweisen, computergesteuert im Raum verfahren und positionieren. Ein solcher motorisiert und computergesteuert bewegbarer C-Bogen wird in der WO 01/50959 offenbart.

Chirurgische Navigationssysteme mit integriertem Computer und Positionserfassungsvorrichtung zur Lagevermessung von im Raum bewegbaren chirurgischen Instrumenten und Geräten werden beispielsweise in der US 5,383,454 BUCHOLZ und der EP 0 359 773 SCHLÖNDORFF offenbart. Im Handel vertrieben werden solche chirurgische Navigationssysteme, beispielsweise unter dem Namen "Surgigate" von der Firma MEDIVISION, Oberdorf, Schweiz. Sie umfassen neben einem Computer mit einem Datenspeicher für Röntgenbilder oder ganze Computertomogramme (CT), welche zur Diagnose und Planung der Operation, vor der Operation oder auch intraoperativ aufgenommen und abgespeichert werden können, mindestens eine Positionserfassungsvorrichtung. Häufig werden optoelektronische Positionserfassungsvorrichtungen eingesetzt, mittels welcher die Lage optischer, an den chirurgischen Instrumenten oder Geräten angebrachter Marker innerhalb eines dreidimensionalen Koordinatensystems im Operationsraum vermessbar ist. Optoelektronische Positionserfassungsvorrichtungen sind beispielsweise unter dem Namen Optotrak 3020 (Hersteller: Northern Digital, Ontario, Canada) im Handel erhältlich.

Als optische Marker werden üblicherweise LED's (Light Emitting Diodes) oder IRED's (Infrared Light Emitting Diodes) eingesetzt, deren Lage innerhalb eines dreidimensionalen Koordinatensystems durch den Sensor, beispielsweise Kameras oder CCD's (Charge-coupled devices) der Positionserfassungsvorrichtung erfassbar ist.

Andere Positionserfassungsvorrichtungen können anstelle von elektromagnetischen Wellen auch akustische Wellen verwenden oder auf der Basis von Magnetfeldern arbeiten.

Zur Definition einer vom C-Bogen einzunehmenden Bildebene oder Bildnormalen kann beispielsweise ein mit Markern versehener Pointer, wie er in der US 5,383,454 BUCHOLZ offenbart ist, vom Operateur mit der Spitze am Körper eines Patienten positioniert werden und der Schaft des Pointers in einer gewählten, der Bildnormalen des aufzunehmenden Röntgenbildes entsprechenden Richtung ausgerichtet werden. Solche Pointer sind beispielsweise unter dem Namen Optotrak Digitizing Probes (Hersteller: Northern Digital, Ontario, Canada) im Handel erhältlich. Die Lage der am Pointer befestigten Marker wird von der Positionserfassungsvorrichtung vermessen und daraus mittels des Computers die Lage und Orientierung der durch die Längsachse des Pointers definierten Geraden innerhalb des dreidimensionalen Koordinatensystems im Raum berechnet. Anschliessend kann der C-Bogen computergesteuert in diejenige Position und Projektion verfahren werden, deren Bildnormale der Längsachse des Pointers entspricht. Nachteilig an der Anwendung soicher Pointer ist, das der Pointer relativ kurz ist und somit der Operateur mit dem Pointer nahe beim Patienten arbeiten muss. Zudem wird die Lage der Bildnormalen relativ zum Patienten mit diesen bekannten Pointern nicht angezeigt, so dass die resultierende Projektion nur grob abschätzbar ist. Die Sichtbarkeit der Marker an solchen kleinen Pointern kann für das Navigationssystem durch den Operateur, den Patienten und durch den C-Bogen, stark eingeschränkt sein oder sogar zeitweilig unterbrochen werden.

Eine Vorrichtung zur Führung eines chirurgischen Instrumentes mit einem Laser, welcher koaxial zur Achse des Führungszylinders einen Laserstrahl emittiert, ist aus der US 5,732,703 KALFAS bekannt. Diese bekannte Vorrichtung umfasst am Führungszylinder angebrachte Marker, deren Position mittels CCD-Kameras innerhalb eines in-situ Koordinatensystemes vermessbar ist. Aus diesen Koordinaten wird der Eintrittspunkt des chirurgischen Instrumentes in den menschlichen Körper sowie die Trajektorie der Achse des Führungszylinders bestimmt. Nachteilig für die Bestimmung der Projektionsebene eines intra-operativ aufzunehmenden Röntgenbildes an dieser bekannten Vorrichtung ist, dass eine Ebene senkrecht zum Laserstrahl vom Chirurgen gedacht werden muss, wodurch leicht Abweichungen auftreten können, welche ein Nachjustieren des Röntgengerätes erfordern.

Hier will die Erfindung Abhilfe schaffen. Der Erfindung liegt die Aufgabe zugrunde, einen Pointer so auszugestalten, dass der Operateur aus einer grösseren Entfernung die Bildnormale anzeigen kann und zugleich eine senkrecht zum Laserstrahl stehende Ebene im Blickfeld hat. Die durch die Erfindung ermöglichten, grösseren räumlichen Abstände zwischen Operateur, Patient und Geräten gestatten eine ungestörte Dektektion der Marker durch die Sensoren der Positionserfassungsvorrichtung. Damit wird erreicht, dass beispielsweise ein computergesteuerter und motorisiert bewegbarer C-Bogen zum Patienten positionierbar ist, ohne dass Operateur und C-Bogen miteinander kollidieren.

Die Erfindung löst die gestellte Aufgabe mit einer Vorrichtung für ein chirurgisches Navigationssystem, welche die Merkmale des Anspruchs 1 aufweist.

Die erfindungsgemässe Vorrichtung für ein chirurgisches Navigationssystem dient zur Definition der Lage einer von einem Operateur bestimmbaren Geraden und einer senkrecht dazu stehenden Ebene innerhalb eines dreidimensionalen Koordinatensystems in einem Operationsraum. Sie umfasst einen Körper, welcher mit mindestens drei Markern versehen ist, wobei die Marker Wellen in den Raum abgeben. Ein Laser ist in den Körper so integriert, dass der Laserstrahl konzentrisch zu einem Zentraistrahl vom Körper weggerichtet emittiert wird. Die Wellenlänge des Laserstrahls liegt im sichtbaren Bereich, so dass der Operateur an einem zu behandelnden Patienten durch entsprechendes Ausrichten der Vorrichtung und des Laserstrahles eine den Patienten schneidende Gerade im Operationsraum definieren kann, welche beispielsweise der Bildnormalen einer einzustellenden Position und Projektion eines C-Bogens entspricht. Der Laser ist im Körper bezüglich der Marker in einer definierten Position fixiert, so dass die Richtung des Zentralstrahles bezüglich der Lage der Marker definiert ist. Die Lage der Marker wird durch eine zum chirurgischen Navigationssytem gehörende Positionserfassungsvorrichtung vermessen, so dass sich mittels eines ebenfalls zum chirurgischen Navigationssystem zählenden Computers die Lage der Geraden innerhalb eines dreidimensionalen Koordinatensystems im Operationsraum ermitteln lässt und beispielsweise der C-Bogen computergesteuert in die vom Operateur gewünschte Position und Projektion verfahren lässt.

In einer Ausführungsform umfasst die erfindungsgemässe Vorrichtung einen Handgriff, welcher im Falle eines batteriebetriebenen Lasers mit einem Hohlraum zur Aufnahme der Batterien ausgestattet sein kann.

Die Leistung des Lasers liegt im Bereich von 2 mW bis 1 W, vorzugsweise zwischen 2 mW und 25 mW.

Die von den Markern abgegebenen Wellen sind korrespondierend zur eingesetzten Positionserfassungsvorrichtung elektromagnetische oder akustische Wellen. Im Falle von Markern, welche elektromagnetische Wellen abgeben, sind folgende Ausführungsformen der Marker möglich:
- LED (Light Emitting Dioden),
- IRED (Infrared Light Emitting Dioden);
- Optische Reflektoren; oder
- durch eine Lichtquelle gespiesene fiberoptische Lichtleiter.

Im Falle von Markern, welche akustisch Wellen abgeben, sind die Marker als akustische Sender ausgeführt.

In einer Ausführungsform der erfindungsgemässen Vorrichtung ist der Körper als prismatischer oder zylindrischer Stab mit einer Längsachse ausgeführt. Der Laser ist im Stab vorzugsweise so integriert, dass der Zentralstrahl mit der Längsachse des Stabes zusammenfällt und der Laserstrahl von einem der Stabenden weggerichtet emittiert wird. Drei Marker können nicht-kolinear am Stab angebracht sein oder auch auf einer Geraden, vorzugsweise auf der Längsachse angeordnet sein, wobei zur Feststellung der Emissionsrichtung des Laserstrahles dann die drei Marker in Abständen (A) respektive (B) relativ zueinander angeordnet sind, wobei (A) nicht gleich (B) ist.

In einer anderen Ausführungsform der erfindungsgemässen Vorrichtung ist der Körper als ebene Platte ausgeführt, wobei die Marker vorzugsweise nicht kolinear angeordnet sind und von der Oberseite der Platte weggerichtet Wellen abgeben. Der Laser ist im wesentlichen mittig in die Platte eingefügt, so dass der Laserstrahl von der Unterseite der Platte weggerichtet emittiert wird.

Vorzugsweise werden die Marker in einer Ebene parallel zur Oberseite angeordnet, wobei die Ebene und die Oberfläche zusammenfallen können, und der Laserstrahl senkrecht zu dieser Ebene emittiert wird.

In einer weiteren Ausführungsform der erfindungsgemässen Vorrichtung umfasst der Laser zur Ermöglichung einer Emission des Laserstrahles in bezüglich der Lage der Marker geometrisch definierter Position und Richtung ein der Wellenlänge des Laserstrahls angepasstes Linsensystem. Anstelle des Linsensystem kann auch eine Faseroptik, ein optisches Spiegelsystem oder ein anderes Strahlablenkungssystem eingesetzt werden.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung eine Gerade sowie eine senkrecht dazu stehende Ebene relativ zu einem Patienten im Operationsraum definierbar ist, welche als einfache Anzeige zur computergesteuerten Positionierung eines medizinischen Gerätes, beispielsweise zur Einstellung der Position und Projektion eines C-Bogens dient. Durch die Reichweite des Laserstrahles ist der Operateur beim Anzeigen der Geraden mittels der erfindungsgemässen Vorrichtung im Raum frei beweglich, so dass beispielsweise der C-Bogen ohne Behinderung des Operateurs positionierbar ist.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Darstellung einer Ausführungsform der erfindungsgemässen Vorrichtung; und
Fig. 2 eine Seitenansicht einer weiteren Ausführungsform der erfindungsgemässen Vorrichtung.

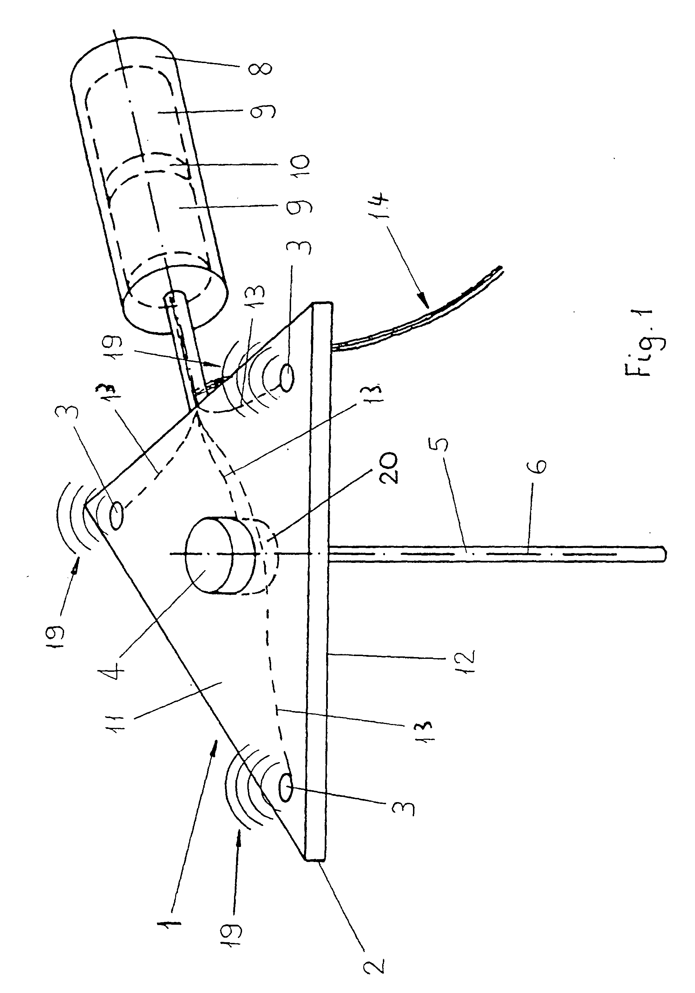

Fig. 1 zeigt eine Ausführungsform der erfindungsgemässen Vorrichtung mit einem Körper 1, welcher als ebene, dreieckförmige Platte mit einer Oberseite 11 und einer Unterseite 12 ausgebildet ist, und einem Laser 4, welcher bezüglich des Plattengrundrisses mittig angeordnet ist und den Laserstrahl 5 von der Unterseite 12 in den Operationsraum emittiert. Der emittierte Laserstrahl 5 weist eine Wellenlänge im sichtbaren Bereich und eine geometrische Zentralachse 6 auf. Damit der Laserstrahl 5 in einer bezüglich der Marker 3 geometrisch definierten Position und Richtung emittierbar ist, umfasst der Laser 4 Mittel 20, welche vorzugsweise als Linsensystem ausführbar sind und im Bereich der Unterseite 12 vorne am Laser 4 angebracht sind. Ebenfalls am Körper 1 angebracht sind drei als LED's ausgeführte Marker 3, wovon je einer in einer der Ecken des Plattengrundrisses so plaziert ist, dass die Wellen (19) in Form von elektromagnetischen Wellen von der Oberseite 11 in den Operationsraum emittiert werden. Seitlich an der Platte ist ein Handgriff 8 befestigt, mittels welchem der Körper 1 manuell frei bewegbar ist. Der Handgriff 8 umfasst einen Hohlraum 10, worin zwei Batterien 9 als Energiequellen für den Laser 4 untergebracht sind. Die Stromversorgung des Lasers 4 erfolgt über ein Kabel 14, welches von den Batterien 9 zum Laser 4 führt und in den Körper 1 integriert ist. Möglich wäre auch eine Kabelführung des Kabels 14 aussen am Körper 1 an der Unterseite 12 oder der Oberseite 11. Auf einem Teil ihrer Länge im Körper 1 integriert sind die Kabel 13, welche die Stromversorgung der Marker 3 gewährleisten. Die Speisung der als LED's ausgeführten Marker 3 erfolgt zentral vom Navigationssystem. Dabei werden die als LED's ausgeführten Marker 3 vom Navigationssystem gepulst angesteuert. Die zeitliche Korrelation zwischen den Steuerpulsen und dem Aufleuchten der einzelnen Marker 3 ermöglicht die Erkennung der mit Markern 3 versehenen chirurgischen Instrumente im Operationsraum durch das Navigationssystem.

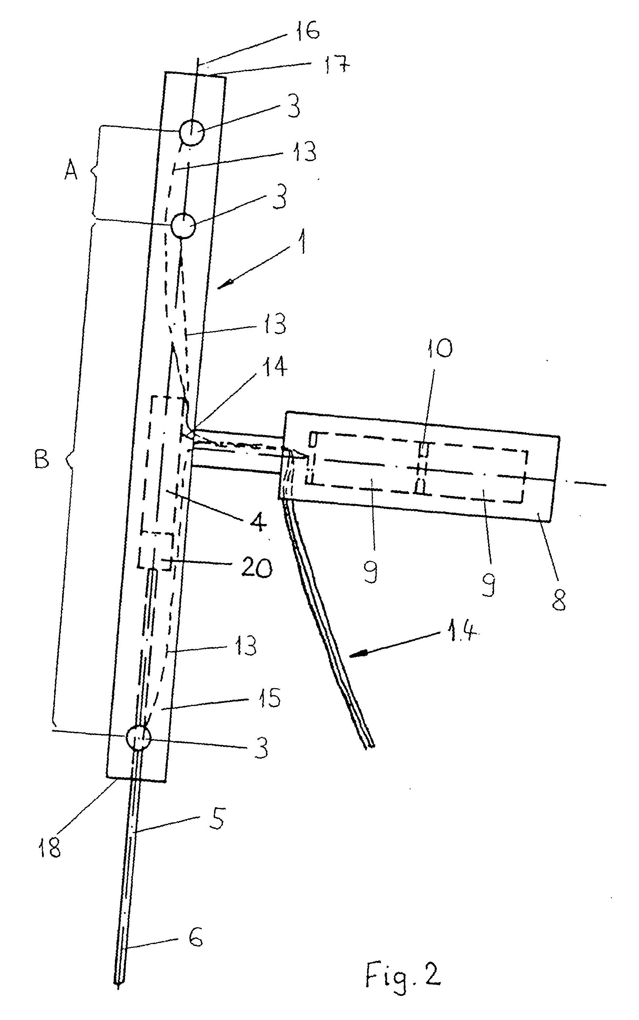

Die in Fig. 2 dargestellte nicht erfindungsgemässe Ausführungsform der Vorrichtung unterscheidet sich von der in Fig. 1 gezeigten Ausführungsform nur darin, dass der Körper 1 als zylindrischer oder prismatischer Stab 15 ausgeführt ist. Der Stab 15 weist eine Zentralachse 16, ein vorderes Ende 18 und ein hinteres Ende 17 auf. Der Laser 4 ist im Stab 15 koaxial zur Zentralachse 16 integriert. Die Mittel 20 zur Emission des Laserstrahls 5 mit bezüglich der Marker 3 geometrisch definierter Position und Richtung, welche auch hier vorzugsweise als Linsensystem ausgeführt werden, sind vorne am Laser 4 in Richtung des vorderen Endes 18 angebracht, so dass der geometrische Zentralstrahl 6 des Laserstrahls 5 mit der Zentralachse 16 zusammenfällt. Rechtwinklig zur Zentralachse 16 am Stab 15 angebracht ist der Handgriff 8 mit dem Hohlraum 10 zur Aufnahme von zwei Batterien 9, welche die Energieversorgung des Lasers 4 übernehmen. Die Stromversorgung des Lasers 4 erfolgt über das Kabel 14, welches im Stab 15 integriert ist und den Laser 4 mit den Batterien 9 verbindet. Drei Marker 3, welche wiederum aus LED bestehen, sind auf einer mit der Zentralachse 16 zusammenfallenden Geraden angeordnet, wobei ein Marker 3 nahe am vorderen Ende 18, ein Marker 3 nahe am hinteren Ende 17 und der dritte Marker 3 dazwischenliegend am Stab 15 angebracht sind. Zwischen dem mittleren Marker 3 und dem Marker 3 nahe am hinteren Ende 17 erstreckt sich ein Abstand A, während sich zwischen dem mittleren Marker 3 und dem Marker 3 am vorderen Ende 18 ein Abstand B erstreckt. Zur Erkennung der Emissionsrichtung des Laserstrahls 5 aus der Lagevermessung der Marker 3 mittels des Navigationssystemes sind die Abstände A und B verschieden ausgestaltet (A < B). Wiederum sind die Kabel 13, welche die Stromversorgung der Marker 3 gewährleisten, auf einem Teil ihrer Länge im Körper 1 integriert und führen vom Handgriff 8 weg zum Navigationssystem.

## Patentansprüche

1. Vorrichtung für ein chirurgisches Navigationssystem zur Definition der Lage einer von einem Operateur bestimmbaren Geraden innerhalb eines dreidimensionalen Koordinatensystems in einem Operationsraum, mit
A) einem Körper (1) mit einer Oberfläche (2) und mindestens drei Wellen (19) abgebenden Markern (3), deren Lage innerhalb des dreidimensionalen Koordinatensystems durch eine zum chirurgischen Navigationssystem gehörende Positionserfassungsvorrichtung bestimmbar ist, wobei
B) der Körper (1) einen Laser (4) umfasst, welcher vom Körper (1) weggerichtet einen Laserstrahl (5) emittiert, welcher einen geometrischen Zentralstrahl (6) und eine Wellenlänge im sichtbaren Bereich aufweist; und
C) durch einen ebenfalls zum chirurgischen Navigationssystem zählenden Computer die Lage des Zentralstrahles (6) bezüglich des dreidimensionalen Koordinatensystems aus der vermessenen Lage der Marker (3) berechenbar ist,
**dadurch gekennzeichnet, dass**
D) der Körper (1) eine ebene Platte mit einer Oberseite (11) und einer Unterseite (12) ist;
E) der Laserstrahl (5) von der Unterseite (12) weggerichtet emittiert wird; und
F) die Marker (3) in einer Ebene parallel zur Oberseite (11) angeordnet sind und dass der Laserstrahl (5) senkrecht zu dieser Ebene emittiert wird.

2. Vorrichtung nach Anspruch 1, **dadurch gekennzeichnet, dass** der Laser (4) Mittel (20) umfasst, wodurch der Laserstrahl (5) bezüglich der Marker (3) in einer geometrisch definierten Position und Richtung emittierbar ist.

3. Vorrichtung nach Anspruch 1 oder 2, **dadurch gekennzeichnet, dass** der Körper (1) einen Handgriff (8) umfasst.

4. Vorrichtung nach einem der Ansprüche 1 bis 3, **dadurch gekennzeichnet, dass** der Laser (4) mittels mindestens einer Batterie (9) betreibbar ist.

5. Vorrichtung nach Anspruch 4, **dadurch gekennzeichnet, dass** der Handgriff (8) einen Hohlraum (10) umfasst, worin die mindestens eine Batterie (9) einsetzbar ist.

6. Vorrichtung nach einem der Ansprüche 1 bis 5, **dadurch gekennzeichnet, dass** der Laser (4) eine Leistung im Bereich von 2 mW bis 1 W, vorzugsweise zwischen 2 mW und 25 mW aufweist.

7. Vorrichtung nach einem der Ansprüche 1 bis 6, **dadurch gekennzeichnet, dass** die Wellen (19) elektromagnetische Wellen sind.

8. Vorrichtung nach Anspruch 7, **dadurch gekennzeichnet, dass** die Marker (3) LED (Light Emitting Dioden) sind.

9. Vorrichtung nach Anspruch 7, **dadurch gekennzeichnet, dass** die Marker (3) IRED (Infrared Light Emitting Dioden) sind.

10. Vorrichtung nach Anspruch 7, **dadurch gekennzeichnet, dass** die Marker (3) optische Reflektoren sind.

11. Vorrichtung nach Anspruch 7, **dadurch gekennzeichnet, dass** die Marker (3) durch eine Lichtquelle gespiesene fiberoptische Lichtleiter sind.

12. Vorrichtung nach einem der Ansprüche 1 bis 6, **dadurch gekennzeichnet, dass** die Wellen (19) akustische Wellen sind.

13. Vorrichtung nach Anspruch 12, **dadurch gekennzeichnet, dass** die Marker (3) akustische Sender sind.

14. Vorrichtung nach einem der Ansprüche 1 bis 13, **dadurch gekennzeichnet, dass** der Körper (1) ein Stab (15) mit einer Längsachse (16), einem vorderen Ende (18) und einem hinteren Ende (17) ist, und der Laser (4) im Stab (15) integriert ist, so dass der Laserstrahl (5) koaxial zur Längsachse (16) von einem Ende (17;18) weggerichtet emittiert wird.

15. Vorrichtung nach einem der Ansprüche 1 bis 14, **dadurch gekennzeichnet, dass** drei Marker (3) auf einer Geraden angeordnet sind, wobei je zwei Marker (3) die Abstände (A) respektive (B) relativ zueinander aufweisen und (A) nicht gleich (B) ist.

16. Vorrichtung nach Anspruch 15, **dadurch gekennzeichnet, dass** die Gerade mit dem Zentralstrahl (6) zusammenfällt.

17. Vorrichtung nach einem der Ansprüche 1 bis 13, **dadurch gekennzeichnet, dass** der Körper (1) eine ebene Platte mit einer Oberseite (11) und einer Unterseite (12) ist, die Marker (3) nicht kolinear angeordnet sind und von der Oberseite (11) Wellen abgeben, und der Laser (4) im wesentlichen mittig in die Platte eingefügt ist und der Laserstrahl (5) von der Unterseite (12) weggerichtet emittiert wird.

18. Vorrichtung nach einem der Ansprüche 1 bis 17, **dadurch gekennzeichnet, dass** die Oberseite (11) die Ebene bildet.

19. Vorrichtung nach einem der Ansprüche 2 bis 18, **dadurch gekennzeichnet, dass** die Mittel (20) ein der Wellenlänge angepasstes Linsensystem umfassen.

20. Vorrichtung nach einem der Ansprüche 2 bis 18, **dadurch gekennzeichnet, dass** die Mittel (20) eine Faseroptik umfassen, welche die Emission des Laserstrahls (5) in einer geometrisch definierten Position und Richtung bezüglich der Marker (3) gestattet.

## Claims

1. A device to be used in combination with a surgical navigation system for defining the position of a straight line determined by an operating surgeon within a three-dimensional coordinate system in an operating theatre, including
A) a body (1) having a surface (2) and at least three markers (3) emitting waves (19) the position of which may be determined within the three-dimensional coordinate system by means of a position detector which is part of the surgical navigation system, whereby
B) the body (1) comprises a laser (4) which emits a laser beam (5), directed away from the body (1), which has a geometrical central beam (6) and a wave length in the visible range; and
C) the position of the central beam (6) relative to the three-dimensional coordinate system may be calculated from the measured positions of the markers (3) by means of a computer which is also part of the surgical navigation system,
**characterised in that**
D) the body (1) is a planar plate with a top surface (11) and a bottom surface (12);
E) the laser beam (5) being emitted in such a way as to be directed away from the bottom surface (12); and
F) the markers (3) are arranged in a plane extending parallel to the top surface (11) and that the laser beam (5) is emitted vertically to said plane.

2. A device as claimed in claim 1, **characterised in that** the laser (4) comprises means (20) permitting the laser beam (5) to be emitted in a geometrically defined position and direction relative to the markers (3).

3. A device as claimed in claim 1 or 2, **characterised in that** the body (1) comprises a handle (8).

4. A device as claimed in any of the claims 1 to 3, **characterised in that** the laser (4) may be operated by at least one battery (9).

5. A device as claimed in claim 4, **characterised in that** the handle (8) comprises a cavity (10) wherein the at least one battery (9) is insertable.

6. A device as claimed in any of the claims 1 to 5, **characterised in that** the laser (4) has a wattage in a range of between 2 mw and 1 W, preferably between 2 mw and 25 mw.

7. A device as claimed in any of the claims 1 to 6, **characterised in that** the waves (19) are electromagnetic waves.

8. A device as claimed in claim 7, **characterised in that** the markers (3) are LEDs (light-emitting diodes).

9. A device as claimed in claim 7, **characterised in that** the markers (3) are IREDs (infrared light-emitting diodes).

10. A device as claimed in claim 7, **characterised in that** the markers (3) are optical reflectors.

11. A device as claimed in claim 7, **characterised in that** the markers (3) are fibre-optic light guides charged by a light source.

12. A device as claimed in any of the claims 1 to 6, **characterised in that** the waves (19) are acoustic waves.

13. A device as claimed in claim 12, **characterised in that** the markers (3) are acoustic emitters.

14. A device as claimed in any of the claims 1 to 13, **characterised in that** the body (1) is a rod (15) having a longitudinal axis (16), a front end portion (18), and a rear end portion (17) and that the laser (4) is integrated into the rod (15), so that the laser beam (5) is emitted coaxially to the central axis (16) in such a way that it is directed away from either one end portion (17; 18).

15. A device as claimed in any of the claims 1 to 14, **characterised in that** three markers (3) are arranged on a straight line, two of said markers (3) being separated from each other by a distance (A) and two markers (3) being separated by a distance (B), the distances (A) and (B) being unequal.

16. A device as claimed in claim 15, **characterised in that** said straight line coincides with the central beam (6).

17. A device as claimed in any of the claims 1 to 13, **characterised in that** the body (1) is a planar plate with a top surface (11) and a bottom surface (12), that the markers (3) are arranged in a non-collinear manner and emit waves from the top surface (11), and that the laser (4) is inserted substantially on centre in the plate, the laser beam (5) being emitted in such a way as to be directed away from the bottom surface (12).

18. A device as claimed in any of the claims 1 to 17, **characterised in that** said plane is formed by the top surface (11).

19. A device as claimed in any of the claims 2 to 18, **characterised in that** the means (20) comprise a system of lenses adapted to the wave length of the laser beam (5).

20. A device as claimed in any of the claims 2 to 18, **characterised in that** the means (20) comprise a fibre-optic appliance permitting the laser beam (5) to be emitted in a geometrically defined position and direction relative to the markers (3).

## Revendications

1. Dispositif destiné à un système de navigation chirurgical permettant de définir, à l'intérieur d'un système de coordonnées tridimensionnel dans une salle d'opération, la position d'une ligne droite pouvant être déterminée par un chirurgien, ledit dispositif comportant
A) un corps (1) avec une surface (2) et au moins trois marqueurs (3) émettant des ondes (19) et dont la position à l'intérieur du système de coordonnées tridimensionnel peut être déterminée par un dispositif de saisie de position faisant partie du système de navigation chirurgical,
B) le corps (1) comprenant un laser (4), lequel émet un rayon laser (5) qui part dudit corps (1) en étant orienté de manière à s'éloigner de celui-ci et qui présente un rayon central géométrique (6) et une longueur d'onde située dans la gamme d'ondes visible; et
C) la position du rayon central (6) par rapport au système de coordonnées tridimensionnel pouvant être calculée par un ordinateur faisant également partie du système de navigation chirurgical à partir de la position mesurée des marqueurs (3),
**caractérisé en ce que**
D) le corps (1) est une plaque plane comportant une face supérieure (11) et une face inférieure (12);
E) le rayon laser (5) est émis à partir de la face inférieure (12) en étant orienté de manière à s'éloigner de celle-ci; et
F) les marqueurs (3) sont disposés dans un plan situé parallèlement à la face supérieure (11) et **en ce que** le rayon laser (5) est émis perpendiculairement à ce plan.

2. Dispositif selon la revendication 1, **caractérisé en ce que** le laser (4) comprend des moyens (20) grâce auxquels le rayon laser (5) peut être émis dans une position et une direction définies géométriquement par rapport aux marqueurs (3).

3. Dispositif selon la revendication 1 ou 2, **caractérisé en ce que** le corps (1) comprend une poignée (8).

4. Dispositif selon l'une des revendications 1 à 3, **caractérisé en ce que** le laser (4) peut fonctionner au moyen d'au moins une pile (9).

5. Dispositif selon la revendication 4, **caractérisé en ce que** la poignée (8) comprend une cavité (10) dans laquelle les piles - dont il existe au moins une - peuvent être insérées.

6. Dispositif selon l'une des revendications 1 à 5, **caractérisé en ce que** le laser (4) présente une puissance située entre 2 mW et 1 W, de préférence entre 2 mW et 25 mW.

7. Dispositif selon l'une des revendications 1 à 6, **caractérisé en ce que** les ondes (19) sont des ondes électromagnétiques.

8. Dispositif selon la revendication 7, **caractérisé en ce que** les marqueurs (3) sont des DEL (diodes à émission optique).

9. Dispositif selon la revendication 7, **caractérisé en ce que** les marqueurs (3) sont des IRED (diodes à émission infrarouge).

10. Dispositif selon la revendication 7, **caractérisé en ce que** les marqueurs (3) sont des réflecteurs optiques.

11. Dispositif selon la revendication 7, **caractérisé en ce que** les marqueurs (3) sont des guides optiques alimentés par une source lumineuse.

12. Dispositif selon l'une des revendications 1 à 6, **caractérisé en ce que** les ondes (19) sont des ondes acoustiques.

13. Dispositif selon la revendication 12, **caractérisé en ce que** les marqueurs (3) sont des émetteurs acoustiques.

14. Dispositif selon l'une des revendications 1 à 13, **caractérisé en ce que** le corps (1) est une tige (15) comportant un axe longitudinal (16), une extrémité avant (18) et une extrémité arrière (17), et **en ce que** le laser (4) est intégré dans la tige (15), de sorte le rayon laser (5) est émis coaxialement à l'axe longitudinal (16) en partant d'une extrémité (17;18) et en étant orienté de manière à s'éloigner de celle-ci.

15. Dispositif selon l'une des revendications 1 à 14, **caractérisé en ce que** trois marqueurs (3) sont disposés sur une ligne droite, les trois marqueurs (3) étant séparés respectivement par les distances (A) et (B), (A) n'étant pas égal à (B).

16. Dispositif selon la revendication 15, **caractérisé en ce que** ladite ligne droite coïncide avec le rayon central (6).

17. Dispositif selon l'une des revendications 1 à 13, **caractérisé en ce que** le corps (1) est une plaque plane comportant une face supérieure (11) et une face inférieure (12), **en ce que** les marqueurs (3) sont disposés de manière non-colinéaire et émettent des ondes partant de la face supérieure (11), et **en ce que** le laser (4) est inséré essentiellement au centre de la plaque, et **en ce que** le rayon laser (5) est émis en partant de la face inférieure (12) et orienté de manière à s'éloigner de celle-ci.

18. Dispositif selon l'une des revendications 1 à 17, **caractérisé en ce que** ledit plan est formé par la face supérieure (11).

19. Dispositif selon l'une des revendications 2 à 18, **caractérisé en ce que** les moyens (20) comprennent un système de lentilles adapté à la longueur d'onde utilisée.

20. Dispositif selon l'une des revendications 2 à 18, **caractérisé en ce que** les moyens (20) comprennent un dispositif basé sur l'optique des fibres qui permet d'émettre le rayon laser (5) dans une position et une direction définies géométriquement par rapport aux marqueurs (3).
